# EUROPEAN PATENT SPECIFICATION

(11) **EP 0 444 240 B1**
(45) Date of publication and mention of the grant of the patent: **26.06.1996**
(21) Application number: 90120292.9
(22) Date of filing: 23.10.1990
(51) Int. Cl.: G01N 33/50, G01N 33/49

(54) **A method for classifying leukocytes and a reagent used therefor**
Verfahren und Reagenz zur Leukozytenklassifikation
Méthode et réactif pour classifier les leucocytes

(30) Priority: 01.03.1990 JP 50814/90
(43) Date of publication of application: 04.09.1991
(73) Proprietor: TOA MEDICAL ELECTRONICS CO., LTD., Kobe-shi Hyogo-ken (JP)
(72) Inventor: Tsujino, Yukio, Kobe-shi, Hyogo-ken (JP); Ohmi, Hitomi, Akashi-shi, Hyogo-ken (JP); Hamaguchi, Yukio, Akashi-shi, Hyogo-ken (JP)
(74) Representative: Hansen, Bernd, Dr. Dipl.-Chem.

(56) References cited:
- EP-A- 0 177 137
- EP-A- 0 316 453
- EP-A- 0 325 710
- WO-A-88/07187
- US-A- 4 286 963

## Description

The present invention relates to methods for classifying blood cells in the practice of clinical testing and, more particularly, to methods for classifying, discriminating and counting leukocytes in a blood sample and reagents used in the methods.

Leukocytes in the peripheral blood of normal subjects can be classified as being of five types consisting of lymphocytes, monocytes, neutrophils, eosinophils, and basophils. Different leukocyte types have different functions and counting of leukocytes in the blood according to their type will provide valuable information for diagnostic purposes. For instance, an increase in the number of neutrophils is associated with such diseases as inflammations, myocardial infraction and leukemia, and a decrease in their number is associated with viral diseases, hypoplastic anemia, agranulocytosis, etc. On the other hand, an increase in the number of eosinophils is found in such diseases as parasitosis, Hodgkin's disease and allergosis. An increased number of monocyte occurs either during the convalescence period of patients suffering from infectious diseases or in such diseases as monocytic leukemia.

Lymphocytes, monocytes neutrophils, eosinophils and basophils are normal cells present in blood. In certain blood diseases, however, cells other than these cells, that is, abnormal cells appear in the peripheral blood. For example, blast cell appear in the peripheral blood of a patient with leukemia.

Since abnormal cells include various different kinds of cells, it is clinically important to classify them and to count cells belonging to each abnormal cell group.

Classification and counting of leukocytes have been made most commonly by the differential counting method which is also referred to as the visual counting method which is also referred to as the visual counting method or simply as the manual method. In this method, a blood sample is spread on a glass slide and the blood cells in the smear are fixed and stained for examination by microscopy. The technician identifies the type of individual leukocytes according to their morphological features (e.g., their size, the morphology of their nucleus and cytoplasm, and the presence or absence of granules) or the degree of dye uptake and performs classification and counting of them. Under ordinary circumstances, 100 - 200 leukocytes are usually counted for each sample and the percentage of the total leukocyte count occupied by each type of cell is recorded as a measured value.

The differential counting method has several disadvantages. First, microscopic observation must be preceded by cumbersome procedures for preparing a specimen that involve such steps as smearing a blood sample on a glass slide, fixing the cells and staining them. Secondly, it is difficult for the technician to identify subtle differences between cells by microscopic classification and counting. Thirdly, it is difficult even for a skilled technician to yield consistent counts by the manual methods since aside from the small number of leukocytes computed, the smeared sample often has an uneven distribution of blood cells.

Various methods have been proposed for eliminating these disadvantages of the manual method of leukocyte classification by developing automated techniques and such automated techniques may be roughly divided into two types. The first method consists of recording the images of cells with a video camera or some other suitable imaging device and classifying the leukocytes by means of image processing on a computer. The operating principle of this method is similar to that of the conventional visual counting method but primarily due to the existence of many cells that defy classification by processing with a computer, this method has not yet become an ideal alternative to the manual method. Furthermore, this method is not economically feasible since it requires sophisticated equipment which is large and costly.

The other approach toward automatic classification and counting of leukocytes is based on a flow system. In this method, a blood sample having cells suspended in a diluent is permitted to flow in such a way that the cells will individually (one by one) pass through a constricted detector and leukocyte classification is done by analyzing the signal generated by the detector. This second method of leukocyte counting which makes use of a flow system is further subdivided into two categories.

In a method of the first category, all red cells present in an electrolyte are destroyed with a lyzing agent so that only suspended leukocytes are permitted to flow through an orifice and the change in electrical impedance that occurs at the orifice when each cell passes through it is detected, with the magnitude of the detected signal being used as a basis for classification of leukocytes.

The fist category includes a method comprising applying a high-frequency current to the orifice and detecting the change in permittivity brought about at the orifice when the blood cells pass through it. This method is referred to as the "RF method" and its basic principle is disclosed in Japanese Patent No. 508789 and U.S. Patent No. 3,390,326. According to the RF method, structures of blood cells and properties of substances constructing the cells in addition to sizes of blood cells can be determined.

Moreover, in the first category including a method comprising applying a direct current to the orifice and detecting the change in permittivity caused when the blood cells pass through it. This method is referred to as the "DC method" and is disclosed in Japanese Patent No. 217947 and U.S. Patent No. 2,656,508. In the DC method, magnitudes of detected signals are almost relative to volume of blood cells.

An apparatus is disclosed in Japanese Patent No. 785859 and U.S. Patent No. 3,502,974 for classifying populations of different kinds of particulates in a suspension by using the RF method in combination with the DC method. However, in these patent specifications, no suggestion is given for the possibility of classifying and counting populations of different kinds of leukocytes.

A method of the second category is characterized by the use of a flow cytometer that comprises a light source, a flow cell that permits the blood cells in a sample to flow one by one through a constricted channel, a photometric unit that detects light issuing from each blood cell, and an analyzer for analyzing the detected signals. In this method, the cells in the sample which are stained are illuminated under a light and the fluorescence emitted from the irradiated cells is detected, optionally together with scattered light with leukocyte classification being made in accordance with the intensity of the detected signals.

Methods belonging to the second category require complicated staining procedures and include many practical problems such as use of a complex and expensive apparatus including an optical system.

On the other hand, cytolytic agents used in a method of the first category include known saponins or quaternary ammonium salts. Since, however, saponins do not exhibit stable hemolytic activity and quaternary ammonium salts exhibit strong hemolytic activity which cause significant damage to leukocytes, there has been a problem that leukocytes could be classified into at most three types by using these hemolytic agents.

According to the applicant's invention of PCT/JP88/00514, neither saponin nor quaternary ammonium salts is used as a cytolytic agent, however, a method for classifying normal leukocytes into five types and classifying abnormal cells comprising using polyoxyethylene nonionic or anionic surfactant to hemolyze erythrocytes and to appropriately injure leukocytes in a short time and analyzing the results by combining the RF and DC methods of the first category is provided with.

On the other hand, Japanese Patent Public Disclosure No. 134957/88 (EP Patent Publication No. 259833) discloses a process for classifying leukocytes (a method of the second category) comprising hemolyzing erythrocytes in the first solution which is acidic and hyposmotic, neutralizing the acidity of the first solution and adjusting the osmolarity by using the second solution, and measuring leukocytes by a flow cytometer to classify them. Moreover, Patent Domestic Announcement No. 502931/89 (PCT/US88/00762) discloses a process (a method of the first category) for classifying leukocytes comprising lyzing erythrocytes in the first solution which is acidic and hyposmotic, neutralizing the acidity of the first solution and adjusting the osmolarity by using the second solution, and classifying and counting leukocytes by using the RF and DC methods in combination.

However, ghosts due to erythrocytes (hereinafter referred to as "erythrocyte ghosts") cannot be sufficiently reduced only by hemolyzing erythrocytes in an acidic and hyposmotic solution as conducted in the above two methods. Therefore, when measuring leukocytes by using RF and DC methods, said ghosts and the intensity of the detected signs partially overlap and as a result, cannot be definitely distinguished. Since either of these two methods requires a reagent consisting of two solutions, an automatic blood analyzer wherein such methods are practised necessarily becomes complicated.

Incidentally, although it is described in a reference [Clinical Analysis (Rinsho Kensa) Book, Additional Volume 1, "Pretreatment for Examination of Samples", page 3, published by Kanahara Shuppan] that smear must be prepared within 4 hours after blood gathering, smears are often prepared many hours after blood gathering in a big institution such as a clinical examination center. In such cases, injury of leukocytes increases at time passes and particularly neutrophils and monocytes are remarkably injured. Therefore, results of the classification of leukocytes obtained from blood gathered 24 hours previously are largely different from those of leukocytes obtained immediately after blood gathering.

EP-A-0 325 710 discloses a reagent for measuring leukocytes and hemoglobin in blood which includes certain polyoxyethylene based non-ionic surfactants. The reagent also includes a buffering agent to maintain the pH of the solution in the range 3-11.

WO 88/07187 discloses a lytic reagent for partitioning a whole blood sample into two fractions, an intact leukocyte fraction and a lysed erythrocyte fraction. The pH of the reagent is maintained in the range 2.6-4.0.

EP-A-0 177 137 discloses a reagent for the determination of basophils which causes the lysis of other forms of leukocyte.

Therefore, there has been a need for a method for accurately classifying leukocytes in blood gathered many hours ago.

The object of this invention is to provide a process wherein neither saponin nor quaternary ammonium is used as a cytolytic agent but another cytolytic agent exhibiting stable hemolytic activities is used to hemolyze erythrocytes and to stabilize leukocytes in a short time, wherein erythrocyte ghosts can be distinguished with lymphocytes and normal leukocytes are classified into five types and abnormal cells are classified, and wherein accurate values of the classified leukocytes can be obtained for blood gather many hours ago; and a reagent used therein.

The problems mentioned above can be solved by using the kit of this invention and the process for classifying leukocytes of this invention using said kit.

The present invention provides a kit for enabling leukocytes to be classified and counted by lyzing erythrocytes and acting on leukocytes, which comprises:
(i) a first solution having a pH of 1.5-5.0, and an osmolarity of 10-120 mmol/kg (10-120 mOsm/kg) and containing a polyoxyethylene based nonionic surfactant represented by the general formula:

   R₁-R₂-(CH₂CH₂O)ₙ-H

   wherein R₁ is an alkyl, alkenyl or alkynyl group having 12-22 carbon atoms; R₂ is -O-, 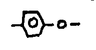 or -COO-; and n is an integer of 20-100; or by the formula 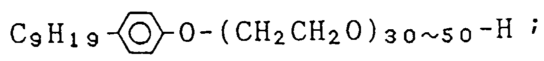 and
(ii) a second solution containing a buffer and an osmolarity adjusting agent, which after addition to the first solution, results in a mixture having a pH of 5.0-12.0 and an osmolarity of 150-2000 mmol/kg (150-2000 mOsm/kg).

Preferably the second solution additionally contains a polyoxyethylene based nonionic surfactant represented by the general formula:

R₁-R₂-(CH₂CH₂O)ₙ-H

wherein R₁ is an alkyl, alkenyl or alkynyl group having 12 - 22 carbon atoms; R₂ is -O-, 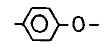 or -COO-; and n is an integer of 20 - 100.

The second solution may also contain at least one solubilizing agent selected from the group consisting of the following five subgroups:

### Subgroup 1 (ureas):

urea
thiourea
1,1-dimethylurea
ethyleneurea
methylurethane
1,3-dimethylurea
urethane (H₂NCOOC₂H₅)

### Subgroup 2:

N-octyl β-D-glucoside CHAPS(3-[(3-chloroamidopropyl)dimethylammonio]-1-propanesulfonate)
CHAPSO(3-[(3-chloroamidopropyl)dimethylammonio]-2-hydroxy-1-propanesulfonate)
MEGA8,9,10(octanoyl-, nonanoyl- or decanoyl-N-methylglucamide)
sucrose monocaprate
N-formylmethylleucylalanine

### Subgroup 3 (guanidines):

guanidine thiocyanate
guanylguanidine
guanidine hydrochloride
guanidine rhodanate
guanidine nitrate
1,1,3,3-tetraguanidine
guanidine carbonate
guanidine phosphate
guanidine sulfate

### Subgroup 4 (cholic acids):

sodium deoxycholate
taurocholic acid
cholic acid

### Subgroup 5 (halogen substituted acetic acids):

sodium trichloroacetate
sodium tribromoacetate
sodium dichloroacetate
sodium dibromoacetate
sodium monochloroacetate
sodium monobromoacetate.

The present invention also provides a method for classifying leukocytes into at least three types; that is, lymphocytes, monocytes and granulocytes by using the above kit and a particulate analysis method selected from a Radio Frequency (RF) method or a Direct Current (DC) method.

The present invention also provides a method for detecting abnormal cells by using the above kit and a particulate analysis method selected from a Radio Frequency (RF) method or a Direct Current (DC) method.

It is well known that erythrocytes are hemolyzed in a hyposmotic solution but the process of this invention is characterized in that erythrocytes can be hemolyzed under hyposmotic (10 - 120 m0sm/kg) and acidic conditions in a solution containing a nonionic surfactant.

If the solution is neutralized, damage to leukocytes becomes severer and erythrocyte ghosts do not sufficiently decrease. On the other hand, if the solution is made alkaline, damage to leukocytes becomes very severe. Therefore, it is essential to hemolyze erythrocytes under acidic condition. Erythrocyte ghosts are decreased only when the acidic and hyposmotic conditions are combined.

Fig. 1 is a chart showing distribution of results obtained by measuring leukocytes immediately after blood gathering using a reagent consisting of one solution only.

Fig. 2 is a chart showing distribution of results obtained by measuring leukocytes 24 hours after blood gathering using a reagent consisting of one solution only.

Fig. 3 is a chart showing distribution of results obtained by measuring leukocytes immediately after blood gathering in a working example using a kit consisting of two solutions.

Fig. 4 is a chart showing distribution of results obtained by measuring leukocytes 24 hours after blood gathering in a working example using a kit consisting of two solutions.

Fig. 5 is a chart indicating distribution of results obtained by measuring blood of a subject suffering from acute myelocytic leukemia in a working example using a kit consisting of two solutions.

Figs. 6 and 7 are charts showing distributions of results obtained by measuring blood merely hemolyzed in an acidic and hyposmotic solution in a reference example. Fig. 6 is a chart relating to measurement immediately after blood gathering and Fig. 7 is one relating to measurement 24 hours after blood gathering.

Fig. 8 is a chart as a reference indicating distribution wherein the addition number (n) of polyethylene is less than that defined in this invention (n=10).

This invention is illustrated by the following examples and non-inventive reference examples but it is not intended to be limited to them.

### Reference Example 1

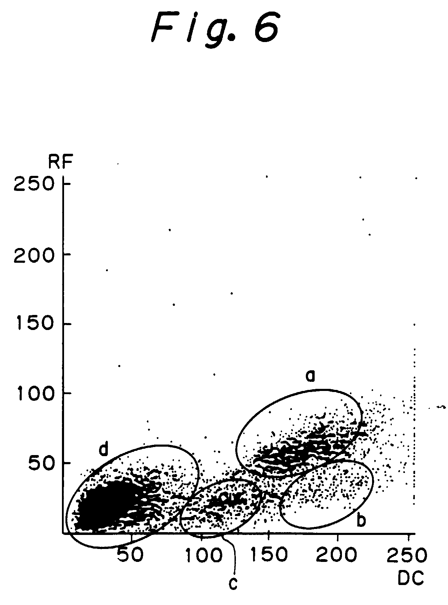

Erythrocytes in a blood sample were hemolyzed and leukocytes in the sample were determined by using a reagent solution wherein the pH was kept at 2.0 and the osmolarity was adjusted to 80 mOsm/kg. The results are indicated in Fig. 6. The transversal axis of the two dimensional distribution shown in Fig. 6 indicates relative signal strength determined by the DC method and the longitudinal axis of said distribution indicates relative signal strength determined by the RF method. Each spot given in Fig. 6 corresponds to each cell which showed each DC signal strength and each RF signal strength.

The signs a - d in Fig. 6 have the following means:
a: granulocytes; b: monocytes; c: lymphocytes;
d: erythrocyte ghosts.

Incidentally, the longitudinal and transversal axes and the signs given in the other figures have the same meanings as in Fig. 6.

Fig. 6 shows the results obtained from blood immediately after gathering. In this figure, distribution of erythrocyte ghosts d and that of lymphocytes c are overlapped in part and distributions of granulocytes a, monocytes b and lymphocytes c are not good in separation (are poorly separated).

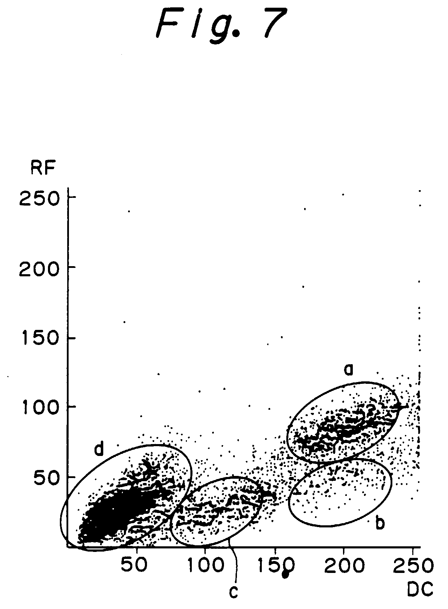

Fig. 7 indicates the results obtained by determination of blood 24 hours after gathering but by using the same reagent as Fig. 6. Distributions of the three kinds of leukocytes are more poorly separated from each other.

### Reference Example 2

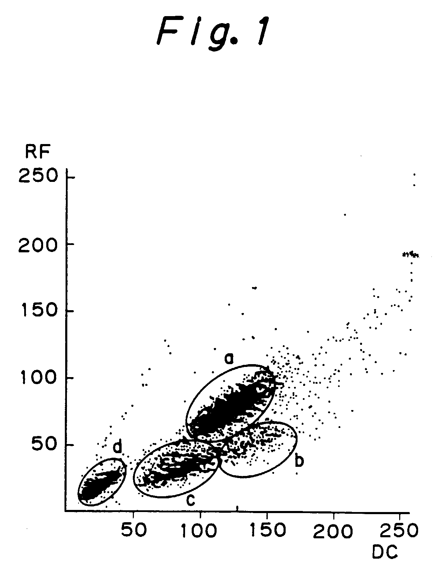

The results obtained when determining blood immediately after gathering by using as a reagent only the first solution of the present kit are indicated in Fig. 1. The pH of the solution was 2.0 and the osmolarity thereof was 80 mOsm/kg. As a polyoxyethylene based nonionic surfactant 1.5 g/ℓ of Emulsit 9 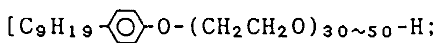 manufactured by Daiichi Kogyo Seiyaku Kabushiki Kaisha] was used. Distribution of erythrocyte ghosts d and that of lymphocytes c are clearly separated without overlapping and distributions of granulocytes a, monocytes c are well separated.

### Reference Example 3

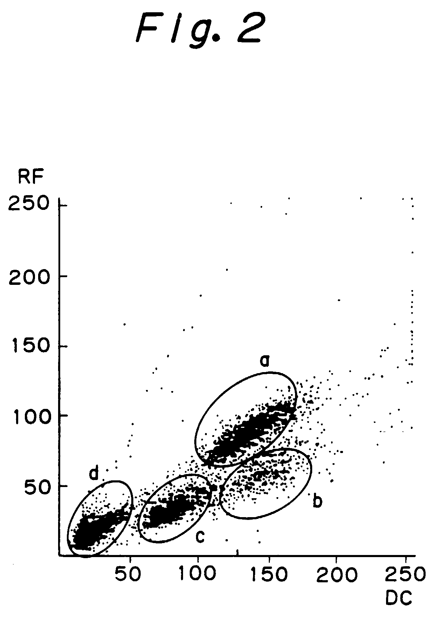

Fig. 2 indicates the results obtained when determining blood 24 hours after gathering by using the same solution as Fig. 1. As shown in Fig. 2, distributions of the three kinds of leukocytes and distribution of erythrocyte ghosts are well separated.

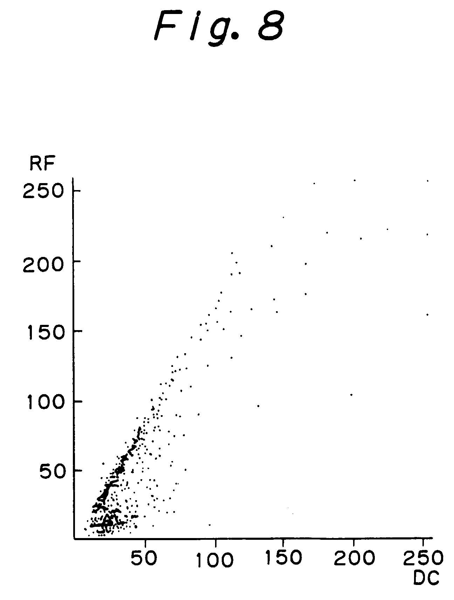

Fig. 8 indicates the results obtained by determining the same blood as in Fig. 1 by using as a polyoxyethylene based nonionic surfactant Actinol L10 [C₁₂H₂₅-O-(CH₂CH₂O)₁₀-H; manufactured by Matsumoto Yushi Seiyaku Kabushiki Kaisha] in place of Emulsit 9. According to Fig. 8, classification and counting of leukocytes became impossible because not only erythrocytes but also leukocytes are destroyed or remarkably contracted. As is already known, the smaller the addition mole number of polyoxyethylene is, the stronger the hemolytic activity thereof is. Actinol L10 wherein n is 10 destroys or contracts not only erythrocytes but also leukocytes.

On the contrary, the larger the addition mole number (n) of polyoxyethylene of the surfactant is, the weaker or zero its hemolytic activity becomes. The surfactant wherein n is large has so weak hemolytic activity that damage to leukocytes is remarkably reduced. Therefore, use of such a surfactant makes possible classification and counting of leukocytes even in blood which was gathered a long time ago. On the other hand, if hemolytic activity of the surfactant is too weak, erythrocyte ghosts cannot be sufficiently reduced so that the ghosts cannot be well distinguished from lymphocytes.

However, surprisingly the inventors found that the erythrocyte ghosts can be sufficiently reduced without causing undesirable leukocytes damage by using the nonionic surfactant wherein the addition mole number of polyoxyethylene is 20 - 100.

Moreover, the inventors also found that the surfactant protects leukocyte membranes and can provide definite separation into three groups of leukocytes: granulocytes a; monocytes b; and lymphocytes c on a two dimensional distribution chart.

An example of said protecting effects is given below:

When hemolyzation was conducted in an acidic and hyposmotic solution, leukocytes are damaged and contracted in about 5 minutes. But when a nonionic surfactant wherein the addition mole number of polyoxyethylene is 20 - 100 is added to the solution, leukocytes do not contract even after 20 minutes has passed.

Kits of the two-solution type provided by the present invention will now be described.

The first solution included in the two-solution type kit as described above is reacted with blood to make erythrocytes hemolyze under an acidic and hyposmotic condition with leukocytes being protected. The second solution containing a buffer and an osmolarity adjusting agent is added to the reaction solution containing the first solution and the blood, whereby the pH and osmolarity of the resulting mixture are adjusted to neutral (pH 5.0 - 12.0) and equivalent to high osmolarity (150 - 2000 mOsm/kg). Thus, the second solution has the effects of adjusting the pH and osmolarity of the reaction solution. By adding the second solution, separation of groups of lymphocytes, monocytes and granulocytes is more improved. whereby analysis of a two-dimensional distribution by a computer to fractionate the distribution into each group is made easy.

Incidentally, separation of said grooups can be still more improved by incorporating a polyoxyethylene based non-ionic surfactant and a solubilizing agent as previously described.

### Example 1

The use of the two-solution kit is illustratively described below.

### Preparation of the first solution

To 1 ℓ of a solution kept at pH 2.0 and adjusted to 80 mOsm/kg with a potassium chloride, hydrochloric acid buffer 1.5 g of Emulsit 9 was added.

### Preparation of the second solution

2 ℓ of a sodium primary phosphate/sodium secondary phosphate buffer was added to 100 g of formalin. After the resulting solution was adjusted to pH 7.5 and 1100 mOsm/kg, 2.5 g of β-phenetyl alcohol as a preservation, 10 g of triethanolamine as an antioxidant and 0.025 g of Emulgen 420 [CH₃(CH₂)₇CH=CH(CH₂)₈-O-(CH₂CH₂O)₁₃H; a polyoxyethylene based nonionic surfactant manufactured by Kao Kabushiki Kaisha] as an agent for constructing erythrocyte ghosts were added to said solution.

### Reaction Condition

To 5 mℓ of the first solution 60 µℓ of blood was added and the mixture was reacted at 33°C for 30 seconds. Thereafter, the second solution was added to the mixture and the resulting mixture was reacted at 33°C for 30 seconds.

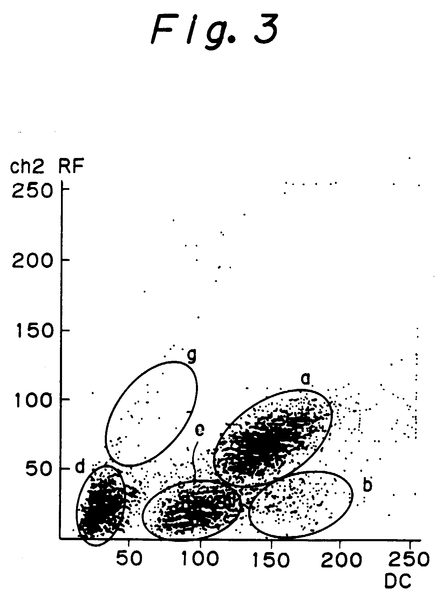

The two dimensional distribution obtained by subjecting blood to the above two step reaction immediately after blood gathering are indicated in Fig. 3. As shown in Fig. 3, erythrocyte ghosts d were sufficiently contracted and granulocytes a, monocytes b and lymphocytes c were well separated. The sign g represents a platelet aggregation.

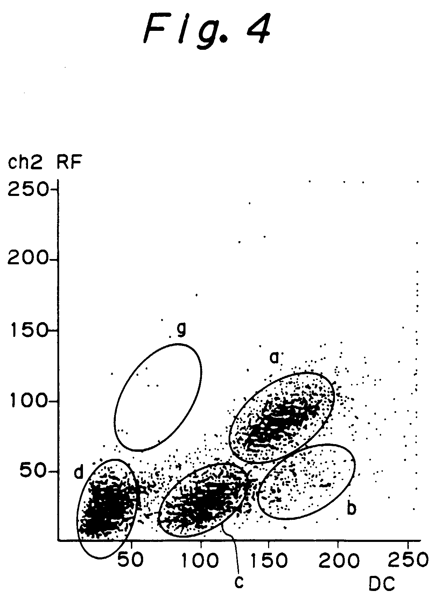

Fig. 4 indicates the results obtained by determining in the same manner as in Fig. 3 the same blood as in Fig. 3 except that 24 hours passed after blood gathering. As clearly shown in Fig. 4, even if determination was carried out 24 hours after blood gathering, substantially the same two dimensional distribution as in Fig. 3 was obtained. This proves that the kit of this invention could realize a stable determination of blood a long time after gathering. This depends on stabilization of leukocytes with the kit of this invention.

Leukocytes were classified into three fractions and, as described in the specification of PCT/JP88/00514, a sample for determination of eosinophils and a sample for determination of basophils were prepared to measure number of eosinophils and that of basophils. By the procedures, the leukocytes could be classified into five types, that is, lymphocytes, monocytes, neutrophils, eosinophils and basophils and cells of each type could be counted to obtain total number of cells of each type of leukocytes and ratios thereof. Coefficients of correlation for ratios of five types of leukocytes obtained by comparing the. results from determination of 20 blood samples according to the above method with the obtained by measuring 200 cells per each of said samples by visual counting method are indicated in Table 1.

**Table 1**

| | |
|---|---|
| lymphocytes | 0.987 |
| monocytes | 0.900 |
| neutrophils | 0.985 |
| eosinophils | 0.979 |
| basophils | 0.525 |

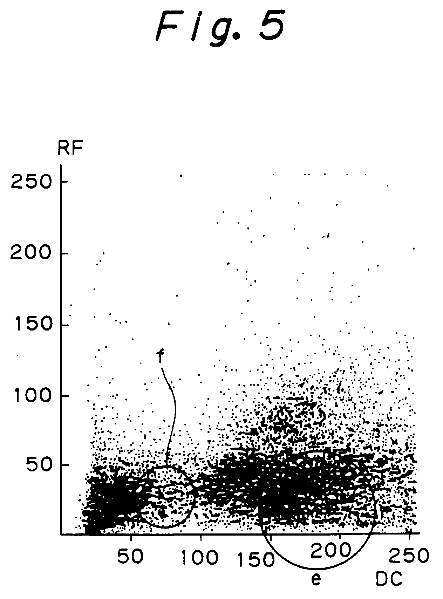

In the same manner, blood of a patient with acute myelocytic leukemia (AML) was determined and the results are indicated as a two-dimensional distribution given in Fig. 5.

Signs e and f mean leukemia cells and nucleate erythrocytes respectively. In Fig. 5, abnormal cells such as leukemia cells appear at the locations at which no cell is present in Figs. 3 and 4. Thus, according to this invention, not only normal cells but also abnormal cells can be detected.

In accordance with the kit and process of this invention, erythrocytes can be hemolyzed by using a cytolytic agent having a stable hemolytic activity; leukocytes can be stabilized in a short time; and erythrocyte ghosts and lymphocytes can be distinguished clearly; and combination of the DC method and the RF method can realize classification of leukocytes blood into five types of normal leukocytes and abnormal cells; and accurate classification values can be obtained even for blood tested a long time after being gathered.

## Claims

1. A kit for enabling leukocytes to be classified and counted by lyzing erythrocytes and acting on leukocytes, which comprises:
(i) a first solution having a pH of 1.5-5.0, and an osmolarity of 10-120 mmol/kg (10-120 mOsm/kg) and containing a polyoxyethylene based nonionic surfactant represented by the general formula:
R₁-R₂-(CH₂CH₂O)ₙ-H
wherein R₁ is an alkyl, alkenyl or alkynyl group having 12-22 carbon atoms; R₂ is -O-, 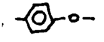 or -COO-; and n is an integer of 20-100; or by the formula 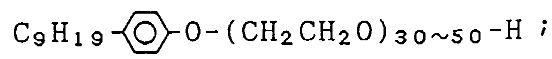 and
(ii) a second solution containing a buffer and an osmolarity adjusting agent, which after addition to the first solution, results in a mixture having a pH of 5.0-12.0 and an osmolarity of 150-2000 mmol/kg (150-2000 mOsm/kg).

2. A kit according to Claim 1, wherein the second solution additionally contains a polyoxyethylene based nonionic surfactant represented by the general formula:
R₁-R₂-(CH₂CH₂O)ₙ-H
wherein R₁ is an alkyl, alkenyl or alkynyl group having 12 - 22 carbon atoms; R₂ is -O-, 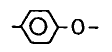 or -COO-; and n is an integer of 20 - 100.

3. A kit according to Claim 1, wherein the second solution contains at least one solubilizing agent selected from the group consisting of the following five subgroups:
- Subgroup 1 (ureas):
urea
thiourea
1,1-dimethylurea
ethyleneurea
methylurethane
1,3-dimethylurea
urethane (H₂NCOOC₂H₅)
Subgroup 2:
N-octyl β-D-glucoside
CHAPS(3-[(3-chloroamidopropyl)dimethylammonio]-1-propanesulfonate)
CHAPSO(3-[(3-chloroamidopropyl)dimethylammonio]-2-hydroxy-1-propanesulfonate)
MEGA8,9,10(octanoyl-, nonanoyl- or decanoyl-N-methylglucamide)
sucrose monocaprate
N-formylmethylleucylalanine
Subgroup 3 (guanidines):
guanidine thiocyanate
guanylguanidine
guanidine hydrochloride
guanidine rhodanate
guanidine nitrate
1,1,3,3-tetraguanidine
guanidine carbonate
guanidine phosphate
guanidine sulfate
Subgroup 4 (cholic acids):
sodium deoxycholate
taurocholic acid
cholic acid
Subgroup 5 (halogen substituted acetic acids):
sodium trichloroacetate
sodium tribromoacetate
sodium dichloroacetate
sodium dibromoacetate
sodium monochloroacetate
sodium monobromoacetate.

4. A method for classifying leukocytes into at least three types; that is, lymphocytes, monocytes and granulocytes by using a kit according to any of claims 1-3 and a particulate analysis method selected from a Radio Frequency (RF) method or a Direct Current (DC) method.

5. A method for detecting abnormal cells by using a kit according to any of claims 1-3 and a particulate analysis method selected from a Radio Frequency (RF) method or a Direct Current (DC) method.

## Patentansprüche

1. Kit zur Klassifizierung und Zählung von Leukozyten unter Lysis von Erythrozyten und Einwirkung auf Leukozyten, umfassend folgende Bestandteile:
(i) eine erste Lösung mit einem pH-Wert von 1,5-5,0 und einer Osmolarität von 10-120 mmol/kg (10-120 mOsm/kg) und mit einem Gehalt an einem nicht-ionogenen Tensid auf Polyoxyethylenbasis der allgemeinen Formel
R₁-R₂-(CH₂CH₂O)ₙ-H
in der R₁ einen Alkyl-, Alkenyl- oder Alkinylrest mit 12-22 Kohlenstoffatomen bedeutet; R₂ die Bedeutungen -O-, 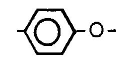 oder -COO- hat; und n eine ganze Zahl von 20-100 ist; oder der Formel 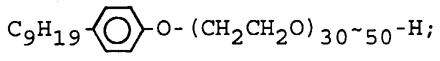 und
(ii) eine zweite Lösung mit einem Gehalt an einem Puffer und einem Mittel zur Einstellung der Osmolarität, das nach Zugabe zur ersten Lösung zu einem Gemisch mit einem pH-Wert von 5,0-12,0 und einer Osmolarität von 150-2000 mmol/kg (150-2000 mOsm/kg) führt.

2. Kit nach Anspruch 1, wobei die zweite Lösung zusätzlich ein nicht-ionogenes Tensid auf Polyoxyethylenbasis der folgenden allgemeinen Formel enthält:
R₁-R₂-(CH₂CH₂O)ₙ-H
in der R₁ einen Alkyl-, Alkenyl- oder Alkinylrest mit 12-22 Kohlenstoffatomen bedeutet; R₂ die Bedeutung -O-, 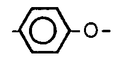 oder -COO- hat; und n eine ganze Zahl von 20-100 ist.

3. Kit nach Anspruch 1, wobei die zweite Lösung mindestens ein Solubilisierungsmittel enthält, das aus folgenden fünf Untergruppen ausgewählt ist:
**Untergruppe 1 (Harnstoffverbindungen):**
Harnstoff
Thioharnstoff
1,1-Dimethylharnstoff
Ethylenharnstoff
Methylurethan
1,3-Dimethylharnstoff
Urethan (H₂NCOOC₂H₅)
**Untergruppe 2:**
N-Octyl-β-D-glucosid
CHAPS (3-[(3-Chloramidopropyl)-dimethylammonio]-1-propansulfonat)
CHAPSO (3-[(3-Chloramidopropyl)-dimethylammonio]-2-hydroxy-1-propansulfonat)
MEGA 8,9,10 (Octanoyl-, Nonanoyl- oder Decanoyl-N-methylglucamid)
Saccharosemonocaprat
N-Formylmethylleucylalanin
**Untergruppe 3 (Guanidine):**
Guanidinthiocyanat
Guanylguanidin
Guanidinhydrochlorid
Guanidinrhodanat
Guanidinnitrat
1,1,3,3-Tetraguanidin
Guanidincarbonat
Guanidinphosphat
Guanidinsulfat
**Untergruppe 4 (Cholsäuren):**
Natriumdesoxycholat
Taurocholsäure
Cholsäure
**Untergruppe 5 (halogensubstituierte Essigsäuren):**
Natriumtrichloracetat
Natriumtribromacetat
Natriumdichloracetat
Natriumdibromacetat
Natriummonochloracetat
Natriummonobromacetat.

4. Verfahren zur Klassifizierung von Leukozyten in mindestens drei Typen, d. h. Lymphozyten, Monozyten und Granulozyten unter Verwendung eines Kits nach einem der Ansprüche 1 bis 3 und unter Anwendung eines speziellen Analyseverfahrens, das unter HF-Verfahren und Gleichstromverfahren ausgewählt ist.

5. Verfahren zum Erfassen von abnormalen Zellen unter Verwendung eines Kits nach einem der Ansprüche 1 bis 3 und unter Anwendung eines speziellen Analysenverfahrens, das unter HF-Verfahren und Gleichstromverfahren ausgewählt ist.

## Revendications

1. Kit permettant de classifier et de compter les leucocytes en lysant les érythrocytes et en agissant sur les leucocytes, lequel comprend:
(i) une première solution d'un pH de 1,5 à 5,0 et d'une osmolarité de 10 à 120 mmoles/kg (10-120 mOsm/kg) contenant un agent tensioactif non-ionique à base de polyoxyéthylène de formule générale:
R₁-R₂-(CH₂CH₂O)ₙ-H
dans laquelle R₁ est un groupe alkyle, alkényle ou alkynyle possédant de 12 à 22 atomes de carbone; R₂ est -O-, 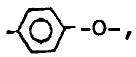 ou -COO-; et n est un nombre entier de 20 à 100; ou de formule 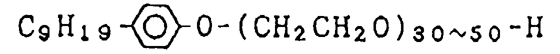 et
(ii) une seconde solution contenant un tampon et un agent permettant d'ajuster l'osmolarité, qui ajouté à la première solution produit un mélange d'un pH de 5,0 à 12,0 et d'une osmolarité de 150-2000 mmoles/kg (150-2000 mOsm/kg).

2. Kit selon la revendication 1 dans lequel la seconde solution contient éventuellement un agent tensioactif non-ionique à base de polyoxyéthylène de formule générale:
R₁-R₂-(CH₂CH₂O)ₙ-H
dans laquelle R₁ est un groupe alkyle, alkényle ou alkynyle possédant de 12 à 22 atomes de carbone; R₂ est -O-, 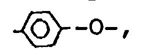 ou -COO-; et n est un nombre entier de 20 à 100.

3. Kit selon la revendication 1 dans lequel la seconde solution contient au moins un agent solubilisant choisi au sein d'un groupe constitué des cinq sous sous-groupes suivants:
Sous-groupe 1 (urées):
urée
thiourée
1,1-diméthylurée
éthyléneurée
méthyluréthane
1,3-diméthylurée
uréthane (H₂NCOOC₂H₅)
Sous-groupe 2:
N-octyl β-D-glycoside
CHAPS(3-[(3-chloroamidopropyl)diméthylammonio}-1-propanesulfonate)
CHAPSO(3-[(3-chloroamidopropyl)diméthylammonio)-2-hydroxy-1-propanesulfonate)
MEGA8, 9, 10(octanoyl-, nonanoyl- ou décanoyl-N-méthylglucamide)
monocaprate de saccharose
N-formylméthylleucylalanine
Sous-groupe 3 (guanidines):
thiocyanate de guanidine
guanylguanidine
chlorhydrate de guanidine
rhodanate de guanidine
nitrate de guanidine
1,1,3,3-tétraguanidine
carbonate de guanidine
phosphate de guanidine
sulfate de guanidine
Sous-groupe 4 (acides choliques):
déoxycholate de sodium
acide taurocholique
acide cholique
Sous-groupe 5 (acides acétiques substitués par un halogène):
trichloroacétate de sodium
tribromoacétate de sodium
dichloroacétate de sodium
dibromoacétate de sodium
monochloroacétate de sodium
monobromoacétate de sodium

4. Procédé de classification des leucocytes en trois types à savoir les lymphocytes, monocytes et granulocytes à l'aide d'un kit selon l'une des revendications 1 à 3 et d'un procédé d'analyse de particules choisi entre une méthode utilisant une Fréquence Radio (FR) et une méthode utilisant un Courant Continu (CC).

5. Procédé de détection de cellules anormales à l'aide d'un kit selon l'une des revendications 1 à 3 et d'un procédé d'analyse de particules choisi entre une méthode utilisant une Fréquence Radio (FR) et une méthode utilisant un Courant Continu (CC).
